# EUROPEAN PATENT SPECIFICATION

(11) **EP 0 529 081 B1**
(45) Date of publication and mention of the grant of the patent: **27.05.1998**
(21) Application number: 92904403.0
(22) Date of filing: 10.02.1992
(51) Int. Cl.: C07D 501/08, C07D 501/59, C25B 3/04, C07D 205/095

(54) **USE OF HALOGENATED BETA-LACTAM COMPOUNDS FOR PRODUCING 3-HYDROXYCEPHEM DERIVATIVES**
VERWENDUNG VON HALOGENIERTEN BETA-LACTAMEN ZUR HERSTELLUNG VON 3-HYDROXYCEPHEM-DERIVATEN
UTILISATION DE COMPOSES BETA-LACTAME HALOGENES POUR LA PRODUCTION DE DERIVES 3-HYDROXYCEPHEM

(30) Priority: 13.03.1991 JP 47084/91
(43) Date of publication of application: 03.03.1993
(73) Proprietor: OTSUKA KAGAKU KABUSHIKI KAISHA, Osaka-shi Osaka-fu 540 (JP)
(72) Inventor: TORII, Shigeru, Akaiwa-gun Okayama 709-08 (JP); TANAKA, Hideo, Okayama-shi Okayama 701-12 (JP); TANIGUCHI, Masatoshi 31-101, Senrimidorigaoka, Toyonaka-shi Osaka 560 (JP); SASAOKA, Mitio, Matsushigecho Itano-gun Tokushima 771-02 (JP); SHIROI, Takashi, Itano-gun Tokushima 771-02 (JP); KAMEYAMA, Yutaka, Okayama-shi Okayama 700 (JP); KIKUCHI, Ryo, Itano-gun Tokushima 771-12 (JP)
(74) Representative: Barz, Peter, Dr.
(86) International application number: JP9200126
(87) International publication number: WO9216532

(56) References cited:
- JP-A- 3 255 066
- JP-A-50 129 590
- JP-A-51 105 051
- JP-A-51 105 088
- JP-A-52 057 162
- JP-A-52 057 188
- Chem. Lett., No. 10, 1990, P. 1867-8.

## Description

The present invention relates to the use of certain halogenated β-lactam compounds for producing 3-hydroxycephem derivatives therefrom.

For the production of 3-hydroxycephem derivatives of the general formula 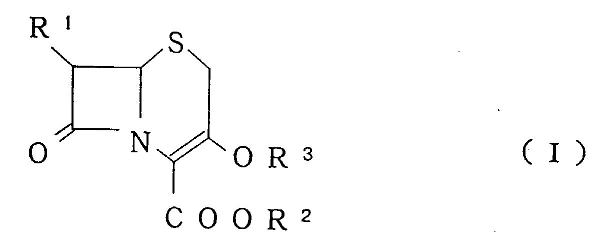 wherein R¹ is an amino group or a protected amino group; R² is a hydrogen atom or a carboxy-protecting group; R³ is a hydrogen atom or a hydroxy-protecting group, typically the process described in Japanese Patent Publication No. 5919/1987 is known.

However, this process is disadvantageous in that since it involves a cyclization reaction with a base and, hence, gives rise to a 2-cephem derivative of the general formula 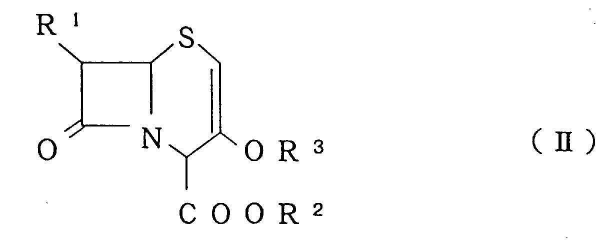 wherein R¹, R² and R³ are as defined above as a byproduct in addition to the aimed at 3-cephem derivative, the desired 3-cephem derivative must be fractionally isolated from the reaction mixture by a purification procedure such as silica gel column chromatography. This means not only an industrial disadvantage process-wise but a low yield of the desired 3-cephem compound.

EP-A-444708 (corresponding to JP-A-3-255066) generically discloses compounds of general formula (III) as shown hereinafter.

Chem. Lett., no. 10, 1990, pages 1867-1868, also discloses compounds falling within said general formula (III) and, in addition, a process wherein said compounds are reacted with certain metallic reducing agents to afford compounds of general formula (I).

US-A-4048162 (corresponding to JP-A-5-257188) and DE-A-2606278 (corresponding to JP-A-51-105051) disclose processes wherein compounds similar to those of general formula (III) below are subjected to ring-closure to prepare 3-hydroxycephem derivatives.

It is an object of the invention is to provide a production process which is free from the disadvantages of the prior art processes and by which 3-hydroxycephem derivatives of general formula (I) can be produced in a safe and expedient manner without using any special reagent and in high yield and purity on a commercial scale.

According to the invention it has been found that certain halogenated β-lactam compounds can be advantageously utilized as starting materials in the production of said 3-hydroxycephem derivatives by electrolytic reduction.

Said halogenated β-lactam compounds can be represented by the following general formula (III): 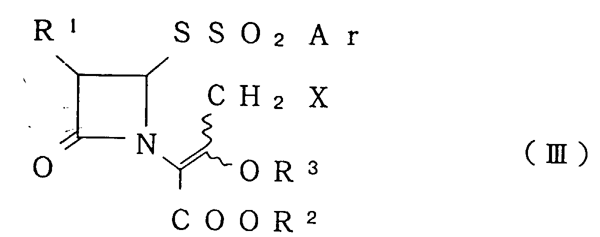 wherein R¹, R² and R³ are as defined hereinbefore; Ar is an aryl group which may be substituted; X is a halogen atom.

The halogenated β-lactam compound of general formula (III) can be advantageously utilized as an intermediate compound for the synthesis of 3-hydroxyceptem derivatives of general formula (I) according to the process of the present invention.

The atomic groups mentioned in this specification specifically mean the following.

The aryl group which may be substituted, designated by the symbol Ar, includes phenyl, naphthyl and so on. The substituent group which may optionally be present on the phenyl or naphthyl group Ar includes, inter alia, halogens (e.g. fluorine, chlorine, bromine and iodine), C₁₋₄ straight-chain or branched alkoxy groups (e.g. methoxy, ethoxy, etc.), C₁₋₄ straight-chain or branched alkylthio groups (e.g. methylthio, ethylthio, etc.), C₁₋₄ straight-chain or branched alkyl groups (e.g methyl, ethyl, etc.), amino, amino substituted by 1 or 2 C₁₋₄ straight-chain or branched alkyl groups (e.g. methylamino, diethylamino, etc.), hydroxyl, acyloxy groups of the formula R⁴COO- (where R⁴ is phenyl, tolyl or a C₁₋₄ straight-chain or branched alkyl group) (e.g. phenylcarbonyloxy, acetyloxy, etc.), acyl groups of the formula R⁴CO- (where R⁴ is as defined above) (e.g. phenylcarbonyl, acetyl, etc.), nitro, cyano, phenyl and so on. The number of such substituents may range from 1 to 5 and is preferably 1, 2 or 3 when the aryl group Ar is phenyl and from 1 to 7 and is preferably 1, 2 or 3 when the aryl group Ar is naphthyl. These substituents may be the same or different.

The protected amino group designated by the symbol R¹ includes not only the various groups mentioned in Theodora W. Greene: Protective Groups in Organic Synthesis, Chapter 7 (pages 218-287) but also, inter alia, phenoxyacetamido, p-methylphenoxyacetamido, p-methoxyphenoxyacetamido, p-chlorophenoxyacetamido, p-bromophenoxyacetamido, phenylacetamido, p-methylphenylacetamido, p-methoxyphenylacetamido, p-chlorophenylacetamido, p-bromophenylacetamido, phenylmonochloroacetamido, phenyldichloroacetamido, phenylhydroxyacetamido, thienylacetamido, phenylacetoxyacetamido, α-oxophenylacetamido, benzamido, p-methylbenzamido, p-methoxybenzamido, p-chlorobenzamido, p-bromobenzamido, phenylglycylamido, amino-protected phenylglycylamido, p-hydroxyphenylglycylamido, and amino- and/or hydroxy-protected p-hydroxyphenylglycylamido. The amino-protecting group on said phenylglycylamido or p-hydroxyphenylglycylamido includes, among others, the various protective groups mentioned in Chapter 7 of the above literature (pages 218-287). The hydroxy-protecting group on said p-hydroxyphenylglycylamido includes, among others, the various protective groups mentioned in Chapter 2 of the above literature (pages 10 to 72).

The carboxy-protecting group R² includes not only the protective groups mentioned in Chapter 5 of the above literature (pages 152-192) but also, inter alia, benzyl, p-methoxybenzyl, p-nitrobenzyl, diphenylmethyl, trichloroethyl, tert-butyl and so on.

The hydroxy-protecting group R³ includes not only the various groups mentioned in Chapter 2 of the above literature (pages 10-72) but also, inter alia, methyl, ethyl, isopropyl, tert-butyl, benzyl, p-methylbenzyl, p-methoxybenzyl, diphenylmethyl, trimethylsilyl, tert-butyldimethylsilyl, triethylsilyl, phenyldimethylsilyl, acetyl and so on.

The halogen atom X includes, inter alia, chlorine, bromine and iodine.

The halogenated β-lactam compound of general formula (III) can be produced by the following or other relevant process.

The compound of general formula (III) wherein R³ is a hydrogen atom can be produced by reacting a β-lactam derivative of the general formula: 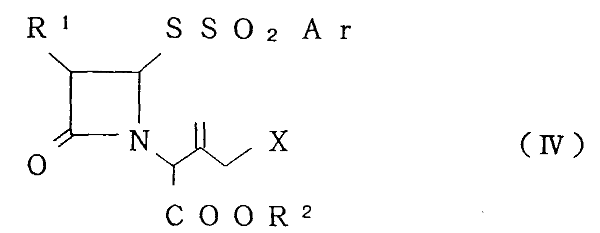 wherein Ar, R¹, R² and X are as defined hereinbefore with ozone to oxidatively eliminate the exomethylene group from said derivative.

This ozonolysis reaction can be carried out by permitting ozone to act on a β-lactam derivative of general formula (IV) in an appropriate solvent to give a peroxide or ozonide which is then reductively decomposed to give the desired compound of general formula (III) wherein R³ is a hydrogen atom.

As regards the conditions of this ozonolysis reaction, the conditions described in the Chemical Society of Japan (ed.): Shin Jikken Kagaku Koza 15, pages 593-603, for instance, can be employed.

Thus, this reaction is conducted in an appropriate solvent. The solvent includes, among others, various alcohols such as methanol, ethanol, propanol, isopropyl alcohol, butanol, tert-butyl alcohol, etc.; lower alkyl esters of lower carboxylic acids, such as methyl formate, ethyl formate, propyl formate, butyl formate, methyl acetate, ethyl acetate, propyl acetate, butyl acetate, methyl propionate, ethyl propionate, etc.; ketones such as acetone, methyl ethyl ketone, methyl propyl ketone, methyl butyl ketone, methyl isobutyl ketone, diethyl ketone, etc.; ethers such as diethyl ether, ethyl propyl ether, ethyl butyl ether, dipropyl ether, diisopropyl ether, dibutyl ether, methylcellosolve, dimethoxyethane, etc.; cyclic ethers such as tetrahydrofuran, dioxane, etc.; nitriles such as acetonitrile, propionitrile, butyronitrile, isobutyronitrile, valeronitrile, etc.; substituted or unsubstituted aromatic hydrocarbons such as benzene, toluene, xylene, chlorobenzene, anisole, etc.; halogenated hydrocarbons such as dichloromethane, chloroform, dichloroethane, trichloroethane, dibromoethane, propylene dichloride, carbon tetrachloride, freons, etc.; aliphatic hydrocarbons such as pentane, hexane, heptane, octane, etc.; cycloalkanes such as cyclopentane, cyclohexane, cycloheptane, cyclooctane, etc.; amides such as dimethylformamide, dimethylacetamide, etc.; dimethyl sulfoxide; and so on. These solvents can be used either independently or in combination. Moreover, the solvents may include water. The solvent is used generally in a proportion of about 10 to about 200 ℓ and preferably in a proportion of about 20 to about 100 ℓ to each kilogram of the compound of general formula (IV).

The reaction temperature for the above reaction is generally about -78°C to about 0°C and preferably about -60°C to about -25°C.

The amount of ozone for use in the above reaction generally need not be more than one equivalent relative to the starting compound (IV). If necessary, however, ozone may be advantageously introduced until disappearance of the starting compound (IV). When the amount of ozone exceeds one equivalent, it is good practice to expell any excess ozone by passing dry nitrogen gas through the reaction mixture before a subsequent treatment is initiated.

The peroxide or ozonide produced in the above reaction is reductively decomposed with a reducing agent which is generally used in organic reactions to give the object compound of general formula (III). The reducing agent or means includes, inter alia, catalytic hydrogenation using a catalyst such as platinum, palladium, nickel, rhodium, etc., phosphorous acid esters, trivalent phosphorus compounds such as triphenylphosphine, dimethyl sulfide and so on.

The resulting compound of general formula (III) wherein R³ is hydrogen may exhibit keto-enol tautomerism.

The compound of general formula (III) wherein R³ is a hydroxy-protecting group can be produced by protecting the hydroxyl function of the above-mentioned compound (general formula (III) wherein R³=H) in the per se known manner. As regards the conditions of the hydroxy-protecting reaction, the various conditions described in Chapter 2 of the literature referred to hereinbefore (pages 10-72), for instance, can be employed.

The halogenated β-lactam compounds (III) of the invention which can be obtained by the above processes can be isolated and purified from the respective reaction mixtures using the conventional purification procedures such as filtration, recrystallization, column chromatography, preparative thin-layer chromatography and so on.

The 3-hydroxycephem derivative of general formula (I) can be easily produced by subjecting the above halogenated β-lactam compound of general formula (III) to electrolytic reduction.

The electrolytic reduction according to this invention is carried out in an organic solvent. The organic solvent mentioned above can be selected from among a diversity of known solvents only if they are able to dissolve the compound of general formula (III) and are inert under the conditions of the reaction. Thus, there may be mentioned, among others, alcohols such as methanol, ethanol, propanol, isopropyl alcohol, butanol, tert-butyl alcohol, etc.; lower alkyl esters of lower carboxylic acids, such as methyl formate, ethyl formate, propyl formate, butyl formate, methyl acetate, ethyl acetate, propyl acetate, butyl acetate, methyl propionate, ethyl propionate, etc.; ketones such as acetone, methyl ethyl ketone, methyl propyl ketone, methyl butyl ketone, methyl isobutyl ketone, diethyl ketone, etc.; ethers such as diethyl ether, ethyl propyl ether, ethyl butyl ether, dipropyl ether, diisopropyl ether, dibutyl ether, methylcellosolve, dimethoxyethane, etc.; cyclic ethers such as tetrahydrofuran, dioxane, etc.; nitriles such as acetonitrile, propionitrile, butyronitrile, isobutyronitrile, valeronitrile, etc.; substituted or unsubstituted aromatic hydrocarbons such as benzene, toluene, xylene, chlorobenzene, anisole, etc.; halogenated hydrocarbones such as dichloromethane, chloroform, dichloroethane, trichloroethane, dibromoethane, propylene dichloride, carbon tetrachloride, freons, etc.; aliphatic hydrocarbons such as pentane, hexane, heptane, octane, etc.; cycloalkanes such as cyclopentane, cyclohexane, chloroheptane, cyclooctane, etc.; amides such as dimethylformamide, dimethylacetamide, etc.; dimethyl sulfoxide and so on. These solvents can be used independently or in combination. Preferably, however, an amine such as dimethylformamide, dimethylacetamide, etc. or a mixture of an amine such as dimethylformamide, dimethylacetamide, etc. with at least one other solvent is employed. The solvent is used in a proportion of generally about 0.5 to about 200 ℓ and preferably about 1 to about 50 ℓ to each 1 kg of the compound of general formula (III).

In conducting this electrolytic reduction according to the invention, a supporting electrolyte is preferably added to the reaction system. The supporting electrolyte includes, among others, metal salts of perchloric acid, such as lithium perchlorate, sodium perchlorate, magnesium perchlorate, etc., ammonium salts of perchloric acid, such as ammonium perchlorate, tetraethylammonium perchlorate, tetrabutylammonium perchlorate, etc., ammonium halides such as ammonium chloride, ammonium bromide, ammonium iodide, tetraethylammonium chloride, tetrabutylammonium bromide, etc., metal borofluorides such as lithium borofluoride, sodium borofluoride, etc., ammonium borofluorides such as tetraethylammonium borofluoride, tetrabutylammonium borofluoride, etc., and various amines such as triethylamine, collidine, lutidine, pyridine, piperidine, N-methylmorpholine, 1,5-diazabicyclo[3.4.0]nonene-5 (DBN), 1,8-diazabicyclo[5.4.0]undecene-7 (DBU) and so on. These supporting electrolytes can be used independently or in combination. Preferably, the amines mentioned above are employed. The amount of the supporting electrolyte generally is about 0.1 to about 100 weight % and preferably about 1 to about 50 weight % in the solvent.

For the electrolytic reduction according to the process of the invention, a diversity of electrodes available for ordinary electrolytic reactions can be selectively employed. Specifically, the positive electrode may be made of, for example, platinum, tin, aluminum, stainless steel, nickel, lead oxide, carbon, iron oxide or titanium, while the negative electrode may be made of, for example, platinum, tin, aluminum, stainless steel, zinc, lead, copper or carbon, and preferably of tin, zinc, lead or copper.

In conducting the electrolytic reduction according to the invention, there are cases in which both current efficiency and reaction efficiency can be improved by adding a halide, inorganic acid salt, organic acid salt or oxide of a metal having a redox potential not higher than that of the substance constituting the negative electrode. This additive includes, among others, the halides (e.g. fluoride, chloride, bromide, iodide), inorganic acid salts (e.g nitrate, sulfate, perchlorate, borate, phosphate, carbonate), organic acid salts (e.g. oxalate, stearate, acetate) and oxides of various metals such as tin, zinc, bismuth, titanium and so on. These additives can be used independently or in combination. The proportion of such additive or additives is generally 0.1 to about 10 mol equivalents and preferably about 0.1 to about 1 mol equivalent relative to the compound of general formula (III). When the above-mentioned additive is employed, addition of said supporting electrolyte may be omitted.

The electrolytic reduction process of this invention may be carried out with the positive and negative electrodes partitioned by a diaphragm. However, the process is characterized in that it can be conducted in a single cell without such partition. The reaction temperature is generally in the range of -10°C to 80°C and preferably 0°C to 50°C.

This electrolytic reaction can be conducted by whichever desired of constant potential (potentiostatic) electrolysis and constant current electrolysis but in terms of the simplicity of equipment required and the expedience of procedure involved, the constant current electrolytic process is preferred. The electrolysis can be carried out with direct current or with alternating current as desired. It may also be carried out by alternating the direction of current every 1 to 30 seconds. The current density is generally 1 to 500 mA/cm² and preferably 1 to 50 mA/cm². While the quantity of electricity is dependent on the electrolytic cell configuration and the species of starting compound (III) and solvent used, it is generally 2 to 10 F/mol and preferably 2 to 5 F/mol. The reaction can be carried to completion when the above quantity of electricity is passed to the cell.

After completion of the above electrolytic reduction, the reaction mixture is concentrated and extracted in the per se known manner to recover the desired 3-hydroxycephem derivative (I) as a substantially pure product. Further purification, if necessary, can be achieved by the conventional purification procedures such as recrystallization, column chromatography and so on.

By using the halogenated β-lactam compound (III) according to this invention, the 3-hydroxycephem derivative of general formula (I) can be produced in a safe and expedient manner with extraordinarily high selectivity, in a high purity requiring virtually no further purification, and in good yield.

### Examples

### Preparation Example 1

In 3 ℓ of ethyl acetate was dissolved 30 g of a compound of general formula (IV) wherein R¹ is phenylacetamido, R² is p-methoxybenzyl, Ar is phenyl and X is chlorine [hereinafter referred to as compound (IVa)] and the solution was cooled to -60°C. Then, ozone generated with an ozone generator was bubbled through this solution. After introduction of one mol equivalent of ozone relative to compound (IVa), 2 mol equivalents, relative to compound (IVa), of dimethyl sulfide was added and the reaction was conducted at 0°C for 1 hour. The reaction mixture was then washed with water, concentrated under reduced pressure and purified by silica gel column chromatography. In this manner, a compound of general formula (III) wherein R¹ is phenylacetamido, R² is p-methoxybenzyl, R³ is hydrogen, Ar is phenyl and X is chlorine [hereinafter referred to as compound (IIIa)] was obtained in a yield of 98%.
NMR (CDCl₃): δppm;
3.60 (s, 2H), 3.80 (s, 3H), 4.23 (ABq, 2H, J=13.0 Hz), 4.73 (dd, 1H, J=5.0 Hz, 7.0 Hz), 5.12 (ABq, 2H, J=13.0 Hz), 5.71 (d, 1H, J=5.0 Hz), 6.27 (d, 1H, J=7.0 Hz), 6.80-8.00 (m, 14H)

### Preparation Example 2

Using a compound of general formula (IV) wherein R¹ is phenylacetamido, R² is diphenylmethyl, Ar is phenyl and X is chlorine [hereinafter referred to as compound (IVb)], the procedure of Preparation Example 1 was followed to give a compound of general formula (III) wherein R¹ is phenylacetamido, R² is diphenylmethyl, R³ is hydrogen, Ar is phenyl and X is chlorine. [hereinafter referred to as compound (III b)].
NMR (CDCl₃): δppm;
3.63 (s, 2H), 4.20 (ABq, 2H, J=13.0 Hz), 4.76 (dd, 1H, J=5.0 Hz, 7.0 Hz), 5.72 (d, 1H, J=5.0 Hz), 6.10 (d, 1H, J=7.0 Hz), 6.85 (s, 1H), 7.05-7.50 (m, 20H)

### Preparation Example 3

Using a compound of general formula (IV) wherein R¹ is phenoxyacetamido, R² is p-methoxybenzyl, Ar is phenyl and X is chlorine, the procedure of Preparation Example 1 was followed to give a compound of general formula (III) wherein R¹ is phenoxyacetamido, R² is p-methoxybenzyl, R³ is hydrogen, Ar is phenyl and X is chlorine.
NMR (CDCl₃): δppm;
3.70 (s, 2H), 3.80 (s, 3H), 4.23 (ABq, 2H, J=13.0 Hz), 4.94 (dd, 1H, J=6.0 Hz, 8.0 Hz), 5.15 (ABq, 2H, J=13.0 Hz), 5.78 (d, 1H, J=6.0 Hz), 6.75-7.95 (m, 14H)

### Preparation Example 4

In 25 ml of methanol was dissolved 2 g of the compound (IIIa) obtained in Preparation Example 1. While the solution was stirred with cooling on an ice bath, a solution of diazomethane in diethyl ether was added dropwise. The dropwise addition was stopped when the color of diazomethane had disappeared. This reaction mixture was concentrated under reduced pressure and the residue was purified by silica gel column chromatography. In this manner, a compound of general formula (III) wherein R¹ is phenylacetamido, R² is p-methoxybenzyl, R³ is methyl, Ar is phenyl and X is chlorine [a mixture of E- and Z-isomers was obtained in a yield of 93%.
NMR (CDCl₃): δppm;
3.65, 3.55 (s, s, 2H), 3.85 (s, 3H), 3.90, 3.91 (s, s, 3H), 4.40 (ABq, 2H, J=18.0 Hz), 4.70 (dd, 1H, J=5.0 Hz, 7.0 Hz), 5.10, 5.25 (s, ABq, 2H, J=24.0 Hz), 5.71, 5.83 (d, d, 1H, J=5.0 Hz), 6.00, 6.13 (d, d, 1H, J=7.0 Hz), 6.80-7.90 (m, 14H)

### Preparation Example 5

The compound (IIIb) obtained in Preparation Example 2 was reacted as in Preparation Example 4 and the reaction product was purified and fractionated by silica gel column chromatography to give isomers, viz. compound (IIIb-1) (E-isomer) and compound (IIIb-2) (Z-isomer). 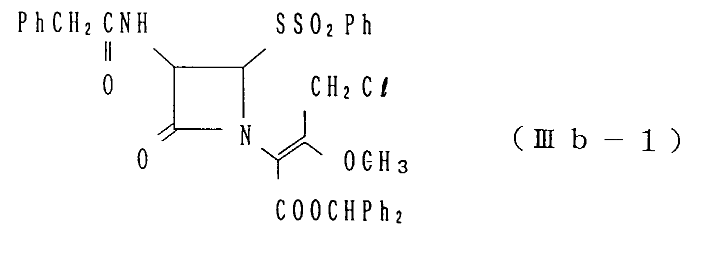 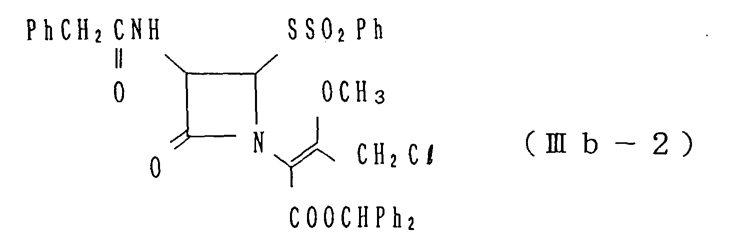 (In the above formulas, Ph means phenyl; the same applies hereinafter)
Physical information on compound (IIIb-1)
NMR (CDCl₃): δppm;
3.65 (s, 2H), 3.94 (s, 3H), 4.38 (ABq, 2H, J=20.0 Hz), 4.70 (dd, 1H, J=5.0 Hz, 7.0 Hz), 5.80 (d, 1H, J=5.0 Hz), 6.05 (d, 1H, J=7.0 Hz), 6.85 (s, 1H), 7.00-7.60 (m, 20H)
Physical information on compound (IIIb-2)
NMR (CDCl₃): δppm;
3.55 (s, 2H), 3.95 (s, 3H), 4.60 (s, 2H), 5.27 (dd, 1H, J=5.0 Hz, 7.0 Hz), 5.69 (d, 1H, J=5.0 Hz), 5.78 (d, 1H, J=7.0 Hz), 6.85 (s, 1H), 7.00-7.70 (m, 20H)

### Preparation Example 6

A reaction vessel was charged with 1 g of the compound (IIIa) obtained in Preparation Example 1 and 2 ml of pyridine and 2 ml of acetic anhydride were added with cooling on an ice bath. The reaction was conducted with stirring at a temperature of 3-5°C for 1 hour. To this reaction mixture was added 2N hydrochloric acid and the mixture was extracted with ethyl acetate. The organic layer was washed successively with 2N hydrochloric acid, saturated aqueous sodium chloride solution, aqueous sodium hydrogencarbonate solution and saturated aqueous sodium chloride solution. The organic layer was dried over anhydrous magnesium sulfate and concentrated under reduced pressure. The residue was purified by silica gel column chromatography. In this manner, a compound of general formula (III) wherein R¹ is phenylacetamido, R² is p-methoxybenzyl, R³ is acetyl, Ar is phenyl and X is chlorine [a mixture of E- and Z-isomers] was obtained in a yield of 90%.
NMR (CDCl₃): δppm;
2.25, 2.00 (s, s, 3H), 3.65, 3.60 (s, s, 2H), 3.80 (s, 3H), 4.50 (ABq, 2H, J=19.0 Hz), 4.75 (dd, 1H, J=8.0 Hz, 14.0 Hz), 5.06 (ABq, 2H, J=20.0 Hz), 5.84, 5.93 (d, d, 1H, J=8.0 Hz), 6.75-7.90 (m, 14H)

### Preparation Example 7

The compound (IIIb) obtained in Preparation Example 2 was reacted as in Preparation Example 6 to give a compound of general formula (III) wherein R¹ is phenylacetamido, R² is diphenylmethyl, R³ is acetyl, Ar is phenyl and X is chlorine [a mixture of E- and Z- isomers];
NMR (CDCl₃): δppm;
2.25, 1.94 (s, s, 3H), 3.60 (s, 2H), 4.75 (dd, 1H, J=5.0 Hz, 7.0 Hz), 5.73, 5.83 (d, d, 1H, J=5.0 Hz), 5.98 (d, 1H, J=7.0 Hz), 7.00-7.70 (m, 20H)

### Preparation Example 8

In 10 ml of dioxane was dissolved 10 g of the compound (IIIa) obtained in Preparation Example 1. To this solution was added 10 g of diphenyldiazomethane and the reaction was conducted at room temperature for 40 minutes and, then, at 40°C for 80 minutes. This reaction mixture was concentrated under reduced pressure and the residue was purified by silica gel column chromatography. In this manner, a compound of general formula (III) wherein R¹ is phenylacetamido, R² is p-methoxybenzyl, R³ is diphenylmethyl, Ar is phenyl and X is chlorine [a mixture of E- and Z- isomers]; was obtained in a yield of 91%.
NMR (CDCl₃): δppm;
3.45, 3.65 (s, s, 2H), 3.80 (s, 3H), 4.10, 4.65 (ABq, ABq, 2H, J=20.0 Hz), 5.15 (ABq, 2H, J=20.0 Hz), 5.85, 5.57 (d, d, 1H, J=5.0 Hz), 5.60, 5.95 (d, d, 1H, J=7.0 Hz), 6.55, 6.60 (s, s, 1H), 6.75-7.80 (m, 24H)

### Preparation Example 9

The compound (IIIb) obtained in Preparation Example 2 was reacted as in Preparation Example 8 and the reaction product was purified and fractionated by silica gel column chromatography to give the isomers, viz. compound (IIIb-3) (E-isomer) and compound (IIIb-4) (Z-isomer). 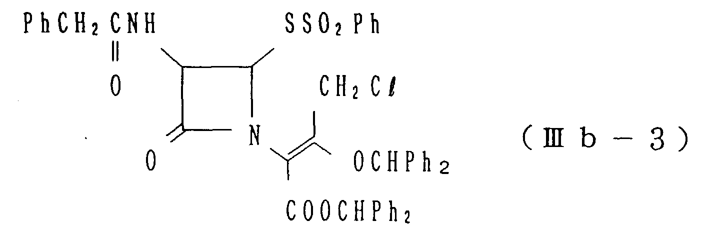 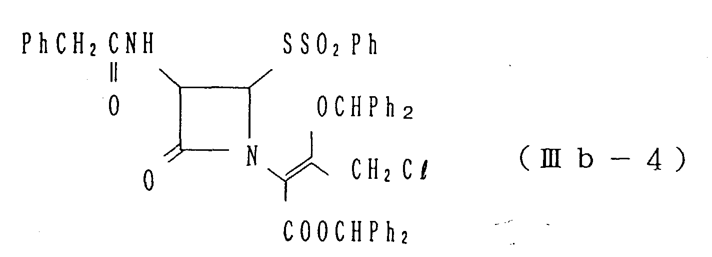 Physical information on compound (IIIb-3)
NMR (CDCl₃): δppm;
3.60 (s, 2H), 4.38 (ABq, 2H, J=20.0 Hz), 4.70 (dd, 1H, J=5.0 Hz, 7.0 Hz), 5.95 (d, 1H, J=7.0 Hz), 6.65 (s, 1H), 6.95-7.65 (m, 30H)
Physical information on compound (IIIb-4)
NMR (CDCl₃): δppm;
3.45 (s, 2H), 4.58 (s, 2H), 5.35 (dd, 1H, J=5.0 Hz, 7.0 Hz), 5.57 (d, 1H, J=5.0 Hz), 5.62 (d, 1H), 5.80 (s, 1H), 6.85-7.75 (m, 30H)

### Example 1

In 3 ml of dimethylformamide was dissolved 100 mg of the compound (IIIa) obtained in Preparation Example 1 and the solution was put in a test tube. To this solution were added 0.38 ml of pyridine and 5 mg of BiCl₃. A couple of tin electrodes (1.5 x 1 cm²) were immersed in this solution and a constant current of 6mA was passed at room temperature for 1 hour and 46 minutes for electrolysis. The reaction mixture was then extracted with ethyl acetate and the extract was washed with 10% hydrochloric acid, dried over anhydrous magnesium sulfate and concentrated under reduced pressure. In this manner, compound (Ia) (R¹=phenylacetamido, R²=p-methoxybenzyl, R³=hydrogen) was obtained in a yield of 92%.

The spectral data on this compound were in complete agreement with those on an authentic sample.

### Example 2

The reaction procedure of Example 1 was repeated except that SnCl₂ was used in lieu of BiCl₃. In this manner, compound (Ia) was obtained in a yield of 95%.

### Example 3

In the procedure of Example 2, the direction of current was alternated every 30 seconds to give compound (Ia) in a yield of 90%.

### Example 4

The electrolysis procedure of Example 3 was repeated except that TiCl₄ was used in lieu of SnCl₂. In this manner, compound (Ia) was obtained in a yield of 88%.

### Example 5

In a mixture of 3 ml of dimethylformamide and 0.38 ml of pyridine was dissolved 100 mg of compound (IIIa). To this was added 100 mg of tetrabutylammonium bromide and a couple of tin electrodes were immersed in the solution. Then, a constant current of 6 mA was passed at room temperature. Electrolysis was carried out by alternating the direction of current every 30 seconds for 2 hours and 50 minutes. The reaction mixture was then treated in the same manner as in Example 1 to give compound (Ia) in a yield of 90%.

## Claims

1. A process for preparing a 3-hydroxycephem derivative having the general formula 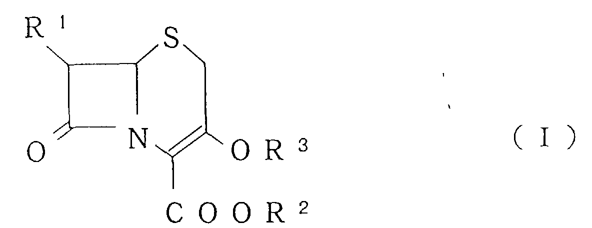 wherein R¹ is an amino group or a protected amino group; R² is a hydrogen atom or a carboxy-protecting group; and R³ is a hydrogen atom or a hydroxy-protecting group,
which comprises subjecting to electrolytic reduction a halogenated β-lactam compound having the general formula 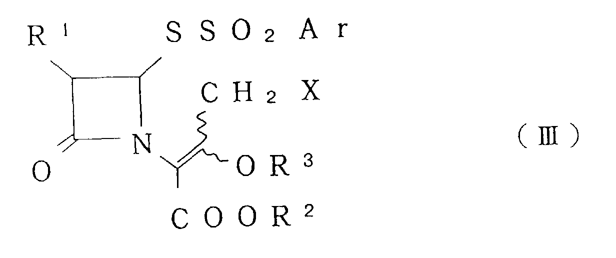 wherein R¹, R² and R³ are as defined above; Ar is an optionally substituted aryl group; and X is a halogen atom.

2. The process of claim 1 wherein a supporting electrolyte is added to the reaction system.

3. The process of claim 1 wherein at least one compound selected from metal perchlorates, ammonium perchlorates, ammonium halides, metal borofluorides, ammonium borofluorides and amines is used as a supporting electrolyte.

4. The process of any one of claims 1-3 wherein platinum, tin, aluminum, stainless steel, nickel, lead oxide, carbon, iron oxide or titanium is used as a positive electrode material and platinum, tin, aluminum, stainless steel, zinc, lead, copper or carbon as a negative electrode material.

5. The process of any one of claims 1-4 wherein said electrolytic reduction is conducted in the presence of a halide, inorganic acid salt, organic acid salt or oxide of a metal having a redox potential not higher than that of the negative electrode material.

## Patentansprüche

1. Verfahren zur Herstellung eines 3-Hydroxycephem-Derivats mit der allgemeinen Formel 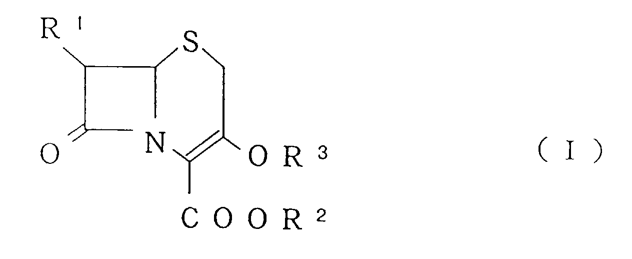 worin R¹ für eine Aminogruppe oder eine geschützte Aminogruppe steht; R² ein Wasserstoffatom oder eine Carboxy-Schutzgruppe darstellt; und R³ ein Wasserstoffatom oder eine Hydroxy-Schutzgruppe ist,
umfassend die elektrolytische Reduktion einer halogenierten β-Lactam-Verbindung mit der allgemeinen Formel 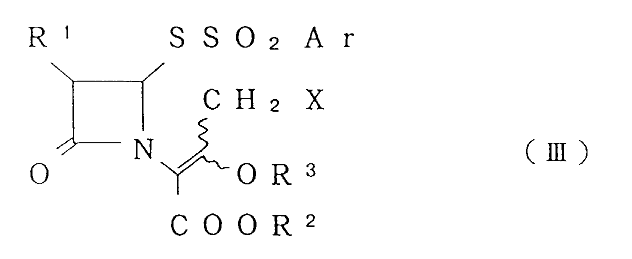 worin R¹, R² und R³ wie oben definiert sind; Ar eine gegebenenfalls substituierte Arylgruppe darstellt; und X ein Halogenatom repräsentiert.

2. Verfahren nach Anspruch 1, in welchem dem Reaktionssystem ein unterstützender Elektrolyt zugesetzt wird.

3. Verfahren nach Anspruch 1, in welchem mindestens eine Verbindung, die aus Metallperchloraten, Ammoniumperchloraten, Ammoniumhalogeniden, Metallborfluoriden, Ammoniumborfluoriden und Aminen ausgewählt ist, als unterstützender Elektrolyt eingesetzt wird.

4. Verfahren nach irgendeinem der Ansprüche 1 - 3, in welchem Platin, Zinn, Aluminium, Edelstahl, Nickel, Bleioxid, Kohlenstoff, Eisenoxid oder Titan als Material für die positive Elektrode eingesetzt werden und Platin, Zinn, Aluminium, Edelstahl, Zink, Blei, Kupfer oder Kohlenstoff als Material für die negative Elektrode eingesetzt werden.

5. Verfahren nach irgendeinem der Ansprüche 1 - 4, in welchem die elektrolytische Reduktion in Anwesenheit eines Halogenids, anorganischen Säuresalzes, organischen Säuresalzes oder Oxids eines Metalles mit einem Redoxpotential, das nicht höher ist als dasjenige des Materials der negativen Elektrode, durchgeführt wird.

## Revendications

1. Procédé de préparation d'un dérivé 3-hydroxycéphem ayant la formule générale : 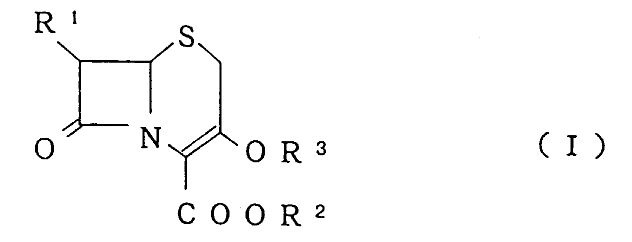 dans laquelle R¹ est un groupe amino ou amino protégé ; R² est un atome d'hydrogène ou un groupe protecteur de groupe carboxy; et R³ est un atome d'hydrogène ou un groupe protecteur de groupe hydroxy,
qui comprend la soumission à une réduction électrolytique d'un composé β-lactame halogéné ayant la formule générale : 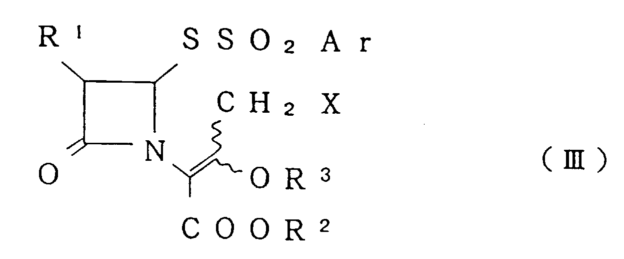 dans laquelle R¹, R², et R³ sont tels que définis ci-dessus ; Ar est un groupe aryle éventuellement substitué ; et X est un atome d'halogène.

2. Procédé selon la revendication 1, dans lequel un électrolyte support est ajouté au système de réaction.

3. Procédé selon la revendication 1, dans lequel au moins un composé choisi parmi les perchlorates métalliques, les perchlorates d'ammonium, les halogénures d'ammonium, les borofluorures métalliques, les borofluorures d'ammonium et les amines, est utilisé comme électrolyte support.

4. Procédé selon l'une quelconque des revendications 1 à 3, dans lequel le platine, l'étain, l'aluminium, l'acier inoxydable, le nickel, l'oxyde de plomb, le carbone, l'oxyde de fer ou l'oxyde de titane est utilisé comme matériau d'électrode positive et le platine, l'étain, l'aluminium, l'acier inoxydable, la zinc, le plomb, le cuivre ou le carbone est utilisé comme matériau d'électrode négative.

5. Procédé selon l'une quelconque des revendications 1 à 4, dans lequel ladite réduction électrolytique est conduite en présence d'un halogénure, d'un sel d'acide minéral, d'un sel organique ou d'un oxyde d'un métal ayant un potentiel redox n'excédant pas celui du matériau de l'électrode négative.
